(19) Europäisches Patentamt
European Patent Office
Office européen des brevets

(11) **EP 4 217 436 B1**

(12) **EUROPÄISCHE PATENTSCHRIFT**

(45) Veröffentlichungstag und Bekanntmachung des Hinweises auf die Patenterteilung:
**17.07.2024   Patentblatt 2024/29**

(21) Anmeldenummer: **22757495.1**

(22) Anmeldetag: **20.07.2022**

(51) Internationale Patentklassifikation (IPC):
**C09J 183/04** (2006.01)    **A61L 15/26** (2006.01)
**C09J 183/06** (2006.01)

(52) Gemeinsame Patentklassifikation (CPC):
(C-Sets verfügbar)
**C09J 183/04; A61L 15/26; A61L 15/58; B32B 5/18;**
**B32B 27/065; B32B 27/283; C09J 183/06;**
B32B 2266/122; B32B 2555/00; C08G 77/20

(Forts.)

(86) Internationale Anmeldenummer:
**PCT/EP2022/070342**

(87) Internationale Veröffentlichungsnummer:
**WO 2023/001887 (26.01.2023 Gazette 2023/04)**

(54) **STRAHLENVERNETZUNG UND STERILISATION VON SILICON**

IRRADIATION CROSSLINKING AND STERILIZATION OF SILICONE

RÉTICULATION PAR IRRADIATION ET STÉRILISATION DE SILICONE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.07.2021   DE 102021118759**

(43) Veröffentlichungstag der Anmeldung:
**02.08.2023   Patentblatt 2023/31**

(73) Patentinhaber: **Paul Hartmann AG**
**89522 Heidenheim (DE)**

(72) Erfinder: **KETTEL, Markus Johannes**
**89520 Heidenheim (DE)**

(74) Vertreter: **Ter Meer Steinmeister & Partner Patentanwälte mbB**
**Nymphenburger Straße 4**
**80335 München (DE)**

(56) Entgegenhaltungen:
**DE-A1- 102016 125 534      US-A1- 2020 146 900**
**US-A1- 2020 222 578**

(52) Gemeinsame Patentklassifikation (CPC): (Forts.)

C-Sets
**A61L 15/26, C08L 75/04;**
**A61L 15/26, C08L 83/04**

**Beschreibung**

**Gebiet der Erfindung**

Die vorliegende Erfindung betrifft ein Verfahren zur Herstellung eines adhäsiven und sterilen Silicons, das adhäsive und sterile Silicon sowie eine Wundauflage umfassend eine Schicht des adhäsiven und sterilen Silicons.

**Hintergrund der Erfindung**

Die vorliegende Erfindung betrifft Wundauflagen, die eine Silicon-Schicht als Adhäsiv enthalten. Wundauflagen müssen während des Herstellungsverfahrens sterilisiert und anschließend steril verpackt werden, um ein keimfreies Milieu zu gewährleisten.

Die Sterilisation von Wundauflagen kann herkömmlich durch Dampfsterilisation, durch Erhitzen in einem Autoklaven oder durch Strahlensterilisation mit ionisierender Strahlung oder durch Gassterilisation stattfinden. Des Weiteren erfolgt die Herstellung unter aseptischen Bedingungen.

Allerdings hat die Dampfsterilisation den Nachteil, dass der verwendete Wasserdampf nicht mit allen Materialien in Wundauflagen kompatibel ist, und insbesondere Hydrogele nicht durch Wasserdampf sterilisiert werden können. So erhöht Wasserdampf unkontrolliert die Feuchtigkeit in Hydrogelen. Gassterilisation hat den Nachteil, dass sich die Gasmoleküle wie z.B. Ethylenoxid in die Struktur der Gele einlagern können.

Die Strahlensterilisation mit ionisierender Strahlung hat den Nachteil, dass diese Methode nicht mit herkömmlichen Silicon-Adhäsiven kompatibel ist. Herkömmliche Silicon-Adhäsive erfahren durch ionisierende Strahlung physikalische Veränderungen und Zersetzung, wodurch das Silicon-Adhäsiv aushärtet und seine adhäsiven Eigenschaften verliert.

US 2020/146900 A1 beschreibt ein Verfahren zur Herstellung eines Wundverbandes, welches folgende Schritte umfasst: Bereitstellen einer mit Öffnungen versehenen Substratschicht; Beschichten der Substratschicht mit einer flüssigen Silikon-Prepolymer-Zusammensetzung; thermisches teilweises Aushärten der Silikon-PrepolymerZusammensetzung, um eine teilweise ausgehärtete Silikonbeschichtung auf dem Substrat zu bilden; Laminieren der beschichteten Substratschicht auf eine Basisschicht, um ein Laminat zu bilden, bei dem die teilweise ausgehärtete Silikonbeschichtung in Kontakt mit einer Oberfläche der Basisschicht steht; Anschließend wird das Laminat einer ionisierenden Strahlung ausgesetzt, um die teilweise ausgehärtete Silikonbeschichtung weiter auszuhärten und die Silikonbeschichtung an die Oberfläche der Basisschicht zu binden. 1

Daher war die Aufgabe der vorliegenden Erfindung die Bereitstellung eines Verfahrens zur Herstellung von sterilem Silicon, welches nach der Strahlensterilisation weiterhin über adhäsive Eigenschaften verfügt.

Eine weitere Aufgabe war die Bereitstellung eines effizienten und schnellen Verfahrens zur Herstellung einer Schicht bzw. Wundkontaktschicht enthaltend adhäsives und steriles Silicon oder einer Wundauflage umfassend eine derartige Schicht bzw. Wundkontaktschicht.

**Zusammenfassung der Erfindung**

Diese Aufgabe wurde überraschenderweise durch ein Verfahren zur Herstellung eines adhäsiven und sterilen Silicons 2 gelöst, umfassend die Bestrahlung eines Silicons 1 mit ionisierender Strahlung mit einer Energiedosis von 5-60 kGy, wobei das Silicon 1

(a) erhältlich ist durch Polykondensation einer Zusammensetzung umfassend Dimethyldichlorsilan und/oder Trimethylmonochlorsilan sowie Einheiten aus der Gruppe bestehend aus

(i) Monomethyltrichlorsilan, und/oder

(ii) einem Element der Gruppe bestehend aus Maleinsäure, Fumarsäure und Trans-3-Hexendisäure sowie Mischungen davon.

Ein so geartetes Silicon 1 lässt sich durch ionisierende Strahlung sterilisieren, ohne sich zu zersetzen und/oder seine adhäsiven Eigenschaften zu verlieren.

Ein weiterer Aspekt der Erfindung betrifft ein adhäsives und steriles Silicon 2 erhältlich oder erhalten durch das oben genannte Verfahren.

Ein weiterer Aspekt der Erfindung betrifft eine Silicon-Schicht enthaltend das adhäsive und sterile Silicon 2 der vorliegenden Erfindung.

Ein weiterer Aspekt der Erfindung betrifft eine Wundauflage umfassend eine Schicht des adhäsiven und sterilen Silicons 2 der vorliegenden Erfindung, wobei die Silicon-Schicht als Wundauflage ausgestaltet sein kann.

Ein weiterer Aspekt der Erfindung betrifft ein Verfahren zur Herstellung der erfindungsgemäßen Wundauflage umfassend

(a) Bereitstellen einer Anordnung umfassend eine Schicht umfassend ein Silicon 1, sowie eine Deckschicht und/oder eine Hydrogelschicht,

(b) optional Verpacken der Anordnung in einer Verpackung,

(c) Bestrahlung der Anordnung mit ionisierender Strahlung zur Härtung und Sterilisierung mit einer Energiedosis E von 5-60 kGy.

Ein weiterer Aspekt der Erfindung betrifft die Verwendung eines oben genannten Silicons 1 in einer Wundkontaktschicht, wobei das Silicon 1 seine adhäsiven Eigenschaften während einer Sterilisierung mit ionisierender Strahlung zur Härtung und Sterilisierung mit einer Energiedosis E von 5-60 kGy beibehält.

## Kurze Beschreibung der Zeichnungen

Fig. 1 beschreibt eine Wundauflage mit einer Silicon-Schicht (10) und einer Hydrogelschicht (12).

In Fig. 2 sind Deckschicht inklusive Verschluss-Tape (26), eine optionale Zwischenschicht (28), Hydrogelschicht (22), Silicon-Schicht (20) und Release Liner (24) zu einer Wundauflage angeordnet.

## Detaillierte Beschreibung der Erfindung

Nachfolgend werden bevorzugte Ausführungsformen der vorliegenden Erfindung detailliert beschrieben. Jede bevorzugte Ausführungsform kann für sich allein oder in Kombination mit weiteren Ausführungsformen verwirklicht werden. Ferner beziehen sich die Erläuterungen zu bevorzugten Ausführungsformen auf alle Aspekte der vorliegenden Erfindung, d. h. auf das Verfahren zur Herstellung eines adhäsiven und sterilen Silicons 2, auf das adhäsive und sterile Silicon 2, auf die Silicon-Schicht enthaltend das adhäsive und sterile Silicon 2, auf die Wundauflage, das Verfahren zur Herstellung der erfindungsgemäßen Wundauflage und auf die Verwendung des Silicons 1 in einer Wundkontaktschicht.

Eine Ausführungsform betrifft ein Verfahren, umfassend die Bestrahlung eines Silicons 1 mit ionisierender Strahlung mit einer Energiedosis von 5-60 kGy, wobei das Silicon 1

(a) erhältlich ist durch Polykondensation einer Zusammensetzung umfassend Dimethyldichlorsilan und/oder Trimethylmonochlorsilan sowie Einheiten aus der Gruppe bestehend aus

(i) Monomethyltrichlorsilan, und/oder

(ii) einem Element der Gruppe bestehend aus Maleinsäure, Fumarsäure und Trans-3-Hexendisäure sowie Mischungen davon.

Die ionisierende Strahlung ist bevorzugt Beta-Strahlung oder Gamma-Strahlung, besonders bevorzugt Beta-Strahlung. Der Fachmann kann ein Dosis-Kalibrierungsmodell für das spezifische Gerät, die Geometrie und die Liniengeschwindigkeit sowie für andere gut bekannte Prozessparameter definieren. Kommerziell erhältliche Geräte zur Erzeugung von Elektronenstrahlen sind ohne weiteres verfügbar. Zum Beispiel ein Modell CB300 Elektronenstrahlerzeugungsgerät (erhältlich von Energy Sciences, Inc. (Wilmington, MA). Im Allgemeinen wird eine Trägerfolie (z. B. Polyester-Terephthalat-Trägerfolie) durch eine Kammer geführt. In der Regel wird die Kammer mit einem Inertgas, z.B. Stickstoff, gespült, während die Proben mit dem Elektronenstrahl gehärtet werden. Es können mehrere Durchläufe durch den Elektronenstrahl-Sterilisator erforderlich sein. Dies umfasst mindestens zwei, mindestens drei, mindestens vier oder mehr Durchläufe. Kommerziell erhältliche Gammabestrahlungsgeräte umfassen Geräte, die häufig für die Gammabestrahlung-Sterilisation von Produkten für medizinische Anwendungen verwendet werden. Kobalt-60-Quellen sind geeignet.

Das Silicon 1 ist durch Polykondensation erhältlich. Die Zusammensetzung kann in Anwesenheit von Wasser hydrolysiert werden, um dann zu polymerisieren.

Der Begriff "Einheiten aus der Gruppe bestehend aus" bezieht sich auf "MonomerEinheiten aus der Gruppe bestehend aus" oder auf ein "Element aus der Gruppe bestehend aus".

Die Begriffe Vinyl-Methyl-Silicon (VMQ), Phenyl-Vinyl-Methyl-Silicon (PVMQ) und Fluor-Vinyl-Methyl-Silicon (FVMQ) sind dem Fachmann geläufig und sind unter anderem kommerziell erhältliche Kautschuke.

VMQ bezeichnet Silicone, die Methyl- und Vinyl-Gruppen aufweisen und z.B. eine Wiederholungseinheit der folgenden Formel (1) umfasst:

$$-[OSi(R^1R^2)]-,$$

wobei $R^1$ Vinyl ist und $R^2$ Methyl ist.

VMQ ist z.B. erhältlich durch Polymerisation einer Zusammensetzung umfassend Vinylmethyldichlorsilan. Dazu kann Vinylmethyldichlorsilan hydrolisiert werden und die Polykondensation der Silanol-Monomere führt zu VMQ.

PVMQ bezeichnet Silicone, die Phenyl-, Methyl- und Vinyl-Gruppen aufweisen. PVMQ kann Phenylmethylsiloxan, Vinylmethylsiloxan und Dimethylsiloxan als Wiederholungseinheiten umfassen und z.B. eine Wiederholungseinheit der folgenden Formel (2) umfassen:

$$-[OSi(R^3R^4)]-,$$

wobei $R^3$ Vinyl oder Phenyl ist und $R^4$ Methyl ist.

Der Begriff "Polymerisation" umfasst die Polykondensation, Polyaddition und/oder radikalische Kettenpolymerisation.

FVMQ bezeichnet Silicone, die Fluor-, Methyl- und Vinyl-Gruppen aufweisen. FVMQ kann Trifluorpropylmethylsiloxan, Vinylmethylsiloxan und Dimethylsiloxane als Wiederholungseinheiten aufweisen. FVMQ kann z.B. eine Wiederholungseinheit der folgenden Formel (3) umfassen:

$$-[OSi(R^5R^6)]-,$$

wobei $R^5$ Vinyl, Fluor oder ein einfach oder mehrfach Fluor-substituiertes $C_1$-$C_3$ Alkan ist und $R^6$ Methyl ist.

Der Begriff "Silicone" umfasst Silicone-Elastomere und Silicone-Kautschuke. Weiterhin sind sowohl RTV ("room-temperature-vulcanizing") als auch HTV ("hightemperature-vulcanizing") Silicone umfasst.

In einer Ausführungsform weist das Silicon 1 einen Vinylgehalt von 0,1-10 Gew.-% auf. Der Vinylgehalt kann bevorzugt mittels NMR-Spektroskopie ermittelt werden.

Hierzu wird als interner Standard Triphenylmethan verwendet. Das Signal des Methinprotons dieses Standards erscheint bei 5,45 ppm und das Signal der Vinylgruppen der Proben erscheint bei 5,7 - 6,0 ppm. Auf der Analysenwaage werden 20 mg in ein kleines Reagenzglas eingewogen. Der interne Standard wird in solchen Mengen eingewogen, dass die charakteristischen Peaks der beiden Substanzen ungefähr die gleichen Integrale ergeben. Dieses Gemisch wird dann in 1 ml Deuterochloroform gelöst und mit Hilfe einer Spritze in ein NMR-Röhrchen überführt. Das Röhrchen wird mit einem Stopfen und einem Parafilm fest verschlossen. Die Proben werden auf einem Bruker-AMX 400 Spektrometer vermessen. Die Auswertung erfolgt über den Vergleich der Integrale der Vinylgruppenpeaks mit dem Integral des Methinprotonenpeaks von Triphenylmethan.

Alternativ kann der Vinylgehalt mittels bromometrischer Titration bestimmt werden (siehe C. Harzdorf. Bestimmung von Si-H und Si-Vinyl in siliciumorganischen Substanzen, Zeitschrift für Analytische Chemie, 276 (1975) 279-283). Hierzu wir eine Probe auf der Analysenwaage in einen Jodzahlkolben eingewogen und in 20 ml $CCl_4$ gelöst. Die Probenmenge wird so gewählt, dass in ihr nicht mehr als 1 mmol an Vinylgruppen vorliegt. 20 ml einer 2,5%igen Quecksilberchloridlösung in Eisessig und 20,0 ml 0,1 M Bromlösung in Eisessig werden dazu gegeben. Die Probe wird 2 Minuten gut durchmischt. Der Kolben wird verschlossen (Schliff mit Eisessig befeuchten) und 30 Minuten im Dunkeln stehen gelassen. Danach werden 2 g KI und 50 ml $H_2O$ hinzugefügt. Das entstandene Jod wird mit einer 0,1 N Natriumthiosulfatlösung titriert. Unter den gleichen Bedingungen wird ein Blindversuch durchgeführt.

In einer Ausführungsform weist das Silicon 1 einen Vinylgehalt von 0.01-100 mmol/g, 0,1-50 mmol/g oder 0.5-10 mmol/g auf.

In einer Ausführungsform weist das Silicon 2 einen Quervernetzungs-Grad von 50-100% auf. In einer bevorzugten Ausführungsform weist das Silicon 2 einen Quervernetzungsgrad von 60-95 % oder 65-80% auf.

In einer Ausführungsform weist das Silicon 1 einen Quervernetzungs-Grad von 0.5-60% auf. In einer bevorzugten Ausführungsform weist das Silicon 1 einen Quervernetzungsgrad von 1-50 %, 5-40 % oder 10-30 % auf.

Der Quervernetzungsgrad bezieht sich auf Gew.-% und kann gemäß folgender Methode bestimmt werden.

Ein Prüfstück (Masse A (mg) des Prüfstücks) wird aus einer stoßdämpfenden Folie so ausgeschnitten, dass die Masse etwa 50 mg beträgt. Das Prüfstück wird in 30 cm$^3$ Chloroform bei 23 °C getaucht, 24 Stunden stehen gelassen und dann durch ein 200-Mesh-Drahtnetz filtriert, um den ungelösten Rückstand auf dem Drahtnetz zu sammeln. Der ungelöste Rückstand wird vakuumgetrocknet und dann wird die Masse B (mg) des ungelösten Rückstandes genau gewogen. Aus dem so erhaltenen Wert wird der Quervernetzungs-Grad nach folgender Gleichung berechnet.

$$\text{Quervernetzungs-Grad (Masse\%)} = (B/A)\ 100$$

Das Silicon 1 kann Vinylgruppen enthalten, welche während der Bestrahlung mit ionisierender Strahlung quervernetzen. Des Weiteren kann die Quervernetzung durch die Zugabe eines Peroxids, wie z.B. Wasserstoffperoxid, beeinflusst werden. In einer Ausführungsform wird das Silicon 1 in Anwesenheit von einer Peroxid-Verbindung, vorzugsweise Wasserstoffperoxid, mit ionisierender Strahlung bestrahlt. Bevorzugt wird 0,01-3 Gew.-% Wasserstoffperoxid verwendet. In einer bevorzugten Ausführungsform kann ganz auf Peroxid-Vernetzer verzichtet werden. Der Begriff Gew.-% bezieht sich hier auf das Gewicht von Wasserstoffperoxid in Bezug auf das Gewicht von Silicon 1 und Wasserstoffperoxid.

In einer Ausführungsform umfasst das Silicon 1 und/oder die Zusammensetzung gemäß

(a) weiterhin ein Element aus der Gruppe bestehend aus
(iii) Divinylether, 1,4-Butandiol-Divinylether, Diethylenglykol-Divinylether und Triethylenglykol-Divinylether sowie Mischungen davon.

Durch den Einbau der Divinylverbindungen aus Gruppe (iii) als zusätzliche Vernetzungsbrücken kann die Quervernetzung und Aushärtung des Silicons 1 beeinflusst werden.

In einer Ausführungsform umfasst die Zusammensetzung gemäß (a) 10-98 Mengen-% Dimethyldichlorsilan und/oder Trimethylmonochlorsilan. In einer bevorzugten Ausführungsform umfasst die Zusammensetzung gemäß (a) 20-98 Mengen-%, 30-95 Mengen-% oder 40-90 Mengen-% Dimethyldichlorsilan und/oder Trimethylmonochlorsilan. Der Begriff Mengen-% bezieht sich auf die Mol-Menge in Bezug auf die Gesamt Mol-Menge der Zusammensetzung gemäß (a).

In einer besonders bevorzugten Ausführungsform umfasst die Zusammensetzung gemäß (a) 10-95 Mengen-%, 50-90 Mengen-% oder 70-80 Mengen-% Dimethyldichlorsilan. In einer weiteren besonders bevorzugten Ausführungsform umfasst die Zusammensetzung gemäß (a) 0,1-40 Mengen-%, 5-30 Mengen-% oder 10-20 Mengen-% Trimethylmonochlorsilan. In einer alternativen Ausführungsform enthält die Zusammensetzung gemäß (a) 0 Mengen-% an Trimethylmonochlorsilan. In anderen Worten, es ist alternativ bevorzugt, dass die Zusammensetzung gemäß (a) kein Trimethylmonochlorsilan enthält

In einer Ausführungsform umfasst die Zusammensetzung gemäß (a) 0,1-10 Mengen-% Monomethyltrichlorsilan, und die Energiedosis beträgt 5-60 kGy, bevorzugt 5-45 kGy, besonders bevorzugt 10-20 kGy. In einer bevorzugten Ausführungsform umfasst die Zusammensetzung gemäß (a) kein Monomethyltrichlorsilan. Monomethylchlorsilan führt zu einer starken Quervernetzung und begünstigt das Aushärten des Silicons 1 während des Bestrahlens. Daher ist es vorteilhaft, wenn die Zusammensetzung gemäß (a) kein oder nur geringe Mengen von 0,1-10 Mengen-% Monomethylchlorsilan umfasst.

In einer Ausführungsform umfasst die Zusammensetzung gemäß (a) 0,1-10 Mengen-% eines Elements der Gruppe bestehend aus Maleinsäure, Fumarsäure und Trans-3-Hexendisäure sowie Mischungen davon, und die Energiedosis beträgt 5-60 kGy, bevorzugt 15-45 kGy, besonders bevorzugt 20-40 kGy.

In einer Ausführungsform umfasst die Zusammensetzung gemäß (a) 0,1-10 Mengen-% eines Elements der Gruppe bestehend aus Divinylether, 1,4-Butandiol Divinylether, Diethylenglykol-Divinylether und Triethylenglykol-Divinylether sowie Mischungen davon, und die Energiedosis beträgt 5-60 kGy, bevorzugt 15-45 kGy, besonders bevorzugt 20-40 kGy.

Die genannte Kombination aus spezifischer Energiedosis und spezifischen Bestandteilen der Zusammensetzung ermöglicht es, die Klebe- und Adhäsionseigenschaften des Silicons 2 gezielt einzustellen. Im Allgemeinen haben stärkere Klebe- und Adhäsionskräfte den Vorteil, dass sie bessere und längere Haftung auf der Haut gewährleisten. Daneben erlauben niedrigere Klebe und Adhäsionskräfte ein leichteres (atraumatisches) Ablösen von der Haut oder können nach dem Aufbringen in ihrer Position korrigiert werden.

Neben dem Einstellen der Energiedosis kann auch die Bestrahlungsrate einen Einfluss auf die Eigenschaften des Silicons 2 haben. In einer bevorzugten Ausführungsform ist die Bestrahlungsrate 0,1-100.0 kGy/min, 1-90 kGy/min, oder 10-60 kGy/min. In einer besonders bevorzugten Ausführungsform ist die Bestrahlungsrate 0,1-100.0 kGy/s, 1-90 kGy/s, oder 10-60 kGy/s. Durch β-Strahlung lassen sich solche hohen Bestrahlungsraten erzielen, wodurch ein besonders schnelles und effizientes Verfahren ermöglicht wird. Dies ist insbesondere bei Serienproduktion im Hochdurchsatzverfahren von Vorteil, wo der Schritt der Vernetzung und/oder Sterilisation die Geschwindigkeit des Gesamtprozesses limitieren kann.

In einer Ausführungsform weist das Silicon 2 eine Adhäsionskraft von 0,4-4 N/cm$^2$ auf und ist gemäß Eur. Ph. 9,00 /2.06.01.00 steril. In einer bevorzugten Ausführungsform beträgt die Adhäsionskraft 0,05-50 N/cm$^2$, 0,8- 3 N/cm$^2$ oder 1-2 N/cm$^2$. Die Adhäsionskraft kann bevorzugt gemäß dem folgenden Testprotokoll gemäßen werden: Es kann ein Zugprüfgerät oder ein ähnliches Gerät verwendet werden, das in der Lage ist, die Klemmbacke mit einer Geschwindigkeit von 300 mm/Minute mit einer Genauigkeit von $\pm 2$ % hin und her zu bewegen. Das Gerät muss in der Lage sein, Kräfte von mindestens 20 Newton mit einer Genauigkeit von $\pm 2$ % zu messen. Eine Platte aus Floatglas muss $25 \pm 0,5$ mm x $30 \pm 2,0$ mm messen und mindestens 3 mm dick sein. An der Unterseite und in der Mitte der Platte ist ein Metallstift angebracht. Die Größe des Stifts sollte so gewählt werden, dass er in die Backe der Zugprüfmaschine eingespannt werden kann. Die Proben sind Streifen von repräsentativen Exemplaren des zu prüfenden Materials. Die Proben müssen 25 mm breit sein und eine Mindestlänge von 175 mm in Maschinenrichtung haben. Der Schnitt muss gerade und sauber sein. Es sollten mindestens 5 Proben pro Muster geprüft werden. Die Prüfung soll bei 23 °C$\pm 2$ °C und 50 $\pm$ 5% r.F. durchgeführt werden. Die Proben oder Prüfstücke müssen vor der Prüfung mindestens 4 Stunden konditioniert werden.

Die beide Enden des Klebestreifens werden gefasst und eine Schlaufe wird gebildet, mit der Klebeseite nach außen, indem die beiden Enden miteinander verbunden werden. Die Schlaufenverbindung wird in die bewegliche Backe der Zugmaschine eingeklemmt und die Schlaufe wird 10 mm frei nach unten hängen gelassen. Die Seiten der Backe müssen vor dem Kleber geschützt werden. Die Glasplatte wird mit ihrem Metallstift in der feststehenden Backe fixiert, wobei die 30-mm-Seite rechtwinklig zum Probenfalz steht. Die Maschine wird gestartet, um die Schleife mit einer Geschwindigkeit von 300 mm/Minute in Kontakt mit der Glasplatte zu bringen. Wenn ein Bereich von ca. 25 mm x 25 mm der Schleife in Kontakt mit der Platte ist, wird die Bewegungsrichtung der Backe umgekehrt, um die Trennung mit 300 mm/Minute einzuleiten. Es ist wichtig, dass die Umkehrung der Bewegungsrichtung so schnell wie möglich erfolgt. Gemessen wird der Maximalwert der Kraft, die erforderlich ist, damit sich die Schleife vollständig von der Platte löst. Die Adhäsion wird als Durchschnittswert der 5 Messungen (ohne Berücksichtigung der anfänglichen Spitze) in Newton (pro Fläche) angegeben.

Falls sich die oben genannte Methode als ungeeignet erweist, kann die Adhäsionskraft alternativ gemäß dem folgenden Testprotokoll gemessen werden.

Es wird eine Ziehvorrichtung verwendet, die in der Lage ist, ein Laminat in einem Winkel von 180° mit einer Geschwindigkeit von 300 mm/Minute und mit einer Toleranz von $\pm$ 2 % zu trennen. Eine Silicon-Schicht mit einer Mindestgröße von 450 mm x 250 mm wird bereitgestellt und mit leichtem Fingerdruck wird ein Streifen Klebeband oder eine Folie mit Klebeflächenmaterial in Maschinenrichtung auf die Silicon-Schicht aufgebracht. Eine Standardrolle wird zweimal mit einer Geschwindigkeit von ca. 10 mm/Sekunde über den Komplex hin und her gerollt. Die Prüfkörper werden zwischen zwei Glas- oder Metallplatten gelegt und anschließend bei 23 °C $\pm$ 2 °C für 20 Stunden unter einem Druck von 6,86 kPa (70 g/cm$^2$) stehen gelassen, um einen guten Kontakt zwischen dem Klebstoff und der Silicon-Schicht zu gewährleisten. Die Prüfkörper werden dann entnommen und für mindestens 4 Stunden bei 23 °C $\pm$ 2 °C und 50 $\pm$ 5 % RH gelagert. Die Prüflinge werden so auf die Prüfmaschine gelegt, dass die Frontplatte in einem 180°-Winkel vom Substrat getrennt werden kann. Die Schicht wird bei Abziehwinkel von 180° mit einer Geschwindigkeit von 300 mm/Minute abgezogen. Die Adhäsion wird als Durchschnittswert der 5 Messungen in Newton (pro Fläche) angegeben.

In einer alternativen Ausführungsform weist das Silicon 2 eine maximale Adhäsionskraft $F_{max}$ von 0,3 bis 4 N, bevorzugt 0,5 bis 3 N, mehr bevorzugt 0,6 bis 2,6 N insbesondere 0,8 bis 1,5 N auf gemessen durch einen Tacktest. Der Tacktest ist im nachstehenden experimentellen Teil beschrieben.

Eine Ausführungsform betrifft adhäsives und steriles Silicon 2 erhältlich durch das erfindungsgemäße Verfahren.

Eine Ausführungsform betrifft eine Silicon-Schicht enthaltend das adhäsive und sterile Silicon 2. Die Schichtdicke der Silicon-Schicht ist bevorzugt 0,1-5 mm.

Eine Ausführungsform betrifft eine Wundauflage umfassend eine erfindungsgemäße Silicon-Schicht, wobei die Silicon-Schicht als Wundkontaktschicht ausgestaltet sein kann. Unter einer Wundauflage wird im Rahmen der vorliegenden Erfindung ein Produkt verstanden, das auf eine Wunde aufgebracht wird in gebrauchsfertiger Form zur Verfügung gestellt wird.

In einer Ausführungsform umfasst die Wundauflage der vorliegenden Erfindung eine Deckschicht, wobei die Deckschicht bevorzugt Polyurethan umfasst.

In einer Ausführungsform umfasst die Wundauflage der vorliegenden Erfindung eine Hydrogelschicht als Wundkontaktschicht enthaltend eine Hydrogelmatrix und/oder einen Hydrogel-Schaumstoff, wobei die Silicon-Schicht als Haftmittel für die Wundauflage ausgestaltet ist. In einer Ausführungsform umfasst die Wundauflage eine Hydrogelschicht auf einer Wundkontaktschicht, wobei die Hydrogelschicht als Hydrogelmatrix ausgebildet sein kann. Der Begriff Hydrogel bezeichnet dabei im Rahmen der Erfindung ein feindisperses System aus mindestens einer festen und einer flüssigen Phase. Diese feste Phase bildet dabei ein schwammartiges, dreidimensionales Netzwerk, dessen Poren durch eine Flüssigkeit (Lyogel) beziehungsweise auch ein Gas (Xerogel) ausgefüllt sind. Beide Phasen durchdringen sich dabei bevorzugt vollständig. Durch Wasseraufnahme kann das dreidimensionale Netzwerk durch Quellen sein Volumen vergrößern, ohne dabei den strukturellen Zusammenhalt zu verlieren. Ein Hydrogel kann bevorzugt aus einem synthetischen oder natürlichen Material, bevorzugt aus einem hydrophilen synthetischen Material, aufgebaut sein. Die Hydrogelschicht kann kontinuierlich oder diskontinuierlich sein. Sie kann beispielsweise vollflächig aufgebracht sein oder Kanäle, Löcher oder anders geformte Öffnungen aufweisen. Bei einer diskontinuierlichen Hydrogelschicht kann eine Vielzahl diskreter Hydrogelelemente umfasst sein, die die Form von Kreisen, Quadraten oder anderen regelmäßigen oder unregelmäßigen Vielecken aufweisen können. In einer erfindungsgemäßen Wundauflage kann eine erfindungsgemäße Hydrogelschicht direkt auf der Silicon-Schicht aufgetragen sein. Ebenso kann die Hydrogelschicht auch mit Hilfe eines Klebstoffes zum besseren Zusammenhalt aufgebracht sein. Zwischen der Silicon-Schicht und der Hydrogelschicht können weitere Schichten angeordnet sein. In einer Ausführungsform ist zwischen der Silicon-Schicht und der Hydrogelschicht eine Schicht umfassend hydrophilen Polyurethan Schaumstoff angeordnet. Denkbar und vorteilhaft ist es darüber hinaus, wenn die Wundauflage wundseitig ein netzförmiges Hydrogel aufweist. Das Hydrogel unterstützt ein feuchtes Wundmilieu und beschleunigt auf diese Weise die Heilung.

Als besonders vorteilhafte Ausführungen haben sich weiterhin Wundauflagen gezeigt, die eine Hydrogelmatrix umfassen, deren Schichtdicke 0,1 bis 5,0 mm aufweist. Insbesondere weist damit eine erfindungsgemäße Wundauflage eine Wundkontaktschicht mit einer Schichtdicke von 0,1 bis 5,0 mm, insbesondere von 0,5 bis 5,0 mm und ganz besonders bevorzugt von 0,5 bis 3,0 mm auf. Wundauflagen mit solchen Schichtdicken zeigen einerseits keine Wundverklebung und andererseits die Fähigkeit, ein von einer Wunde abgegebenes Wundexsudat aufzunehmen und an die absorbierende Schicht weiterzuleiten. Diese Schichtdicken können an jeder Stelle der Wundkontaktschicht gleich sein oder in verschiedenen Bereichen der Wundkontaktschicht verschiedene Werte annehmen.

Weiterhin bevorzugt kann die Hydrogelmatrix Kanäle umfassen, insbesondere konische Kanäle, zum Durchtritt von Flüssigkeiten von der ersten zur zweiten Seite. Hierdurch kann insbesondere ein verbesserter Durchtritt für Wundexsudat bereitgestellt und ein Rückfluss in Richtung Wunde minimiert werden. Hierbei ist besonders bevorzugt vorgesehen, dass die Kanäle einen elliptischen oder einen kreisförmigen Querschnitt aufweisen, d.h. dass die Kanäle eine kreisförmige oder elliptische Öffnung sowohl an der ersten als auch an der zweiten Seite der Hydrogelmatrix aufweisen, wobei die kreisförmige oder elliptische Öffnung auf der ersten und der zweiten Seite verschieden groß sind. Es kann jedoch auch vorgesehen sein, dass die Kanäle einen dreieckigen, rechteckigen, quadratischen, fünfeckigen, sechseckigen oder einen anderen vieleckigen Querschnitt aufweisen. Dabei ist ganz besonders bevorzugt vorgesehen, dass die erste Seite Öffnungen aufweist, die im Vergleich zu der auf der zweiten Seite befindlichen Öffnung größer ist (konische Ausführung).

Gemäß einer Weiterbildung der Erfindung kann auch vorgesehen sein, dass die Wundkontaktschicht oder die Hydrogelmatrix Öffnungen aufweist, die einen Durchmesser von 0,5 bis 5 mm aufweisen. Insbesondere weist die Wundkontaktschicht oder die Hydrogelmatrix Öffnungen auf, die einen Durchmesser von 1 bis 3 mm aufweisen. Ganz besonders bevorzugt weist die Wundkontaktschicht oder die Hydrogelmatrix auf der wundzugewandten ersten Seite Öffnungen auf, die einen Durchmesser von 1 bis 3 mm aufweisen, wobei die zweite Seite der Wundkontaktschicht oder der Hydrogelmatrix in direktem Kontakt mit dem Polyurethanschaum steht.

Die erfindungsgemäßen Wundauflagen sind für die Behandlung von Wunden geeignet. Die vorliegende Erfindung umfasst daher auch Wundauflagen zur Behandlung von Wunden. Insbesondere umfasst die vorliegende Erfindung Wundauflagen zur Behandlung chronischer Wunden wie Dekubitus, Druck-Ulzera, Druckgeschwüre, Ulcus cruris venosum, venöse Ulzera, Ulcus cruris arteriosum, arterielle Ulzera, Wunden infolge von diabetischem Fußsyndrom, neuropathische Ulzera, aber auch Wunden in Folge von Autoimmunerkrankungen oder von Tumoren (exulzierende Tumore) oder von Strahlenschäden bei der Tumortherapie.

Erfindungsgemäße Wundauflagen sind zur phasengerechten Wundtherapie geeignet, insbesondere zur Therapie von Wunden in der Granulationsphase und/oder der Epithelisierungsphase.

In einer Ausführungsform umfasst die Hydrogelmatrix mindestens 3-95 Gew.-%, insbesondere 5-90 Gew.-% Wasser.

Die Hydrogelmatrix kann ein Polyurethan-Polyharnstoff-Copolymer umfassen. Dieses Polyurethan-Polyharnstoff-Copolymer kann dabei insbesondere aus einem Präpolymer mit aliphatischen Diisocyanat-Gruppen und einem Polyamin auf Polyethylenoxidbasis gebildet werden. Insbesondere kann das Polyurethan-Polyharnstoff-Copolymer dabei aus einem Präpolymer mit Isophorondiisocyanat-Enden, einem Polyamin auf Polyethylenoxidbasis und Wasser gebildet werden. Diese Hydrogelmatrices sind besonders gut geeignet, Wasser einzulagern und dieses Wasser an eine Wunde abzugeben.

Weiterhin bevorzugt kann die wasserhaltige Hydrogelmatrix weiterhin mindestens einen mehrwertigen Alkohol aus der Gruppe der zweiwertigen, dreiwertigen, vierwertigen, fünfwertigen oder sechswertigen Alkohole umfassen. Insbesondere kann der Alkohol gewählt werden aus der Gruppe der Glykole, insbesondere Ethylenglykol oder Propylenglykol, sowie Sorbitol oder Glycerin oder Mischungen hiervon. Diese Alkohole sind hervorragend als Feuchtigkeitsspender geeignet und stellen somit für die die Wunde umgebende Haut eine pflegende Komponente dar. Die Hydrogelmatrix oder der Hydrogel-Schaumstoff kann 1-50 Gew.-%, bevorzugt 10 -30 Gew.-%, eines mehrwertigen Alkohols umfassen.

Darüber hinaus kann vorgesehen sein, dass die wasserhaltige Hydrogelmatrix insbesondere mindestens ein Salz umfasst. Insbesondere ist hierbei vorgesehen, dass die Hydrogelmatrix ein anorganisches Salz umfasst. Besonders geeignet sind in diesem Zusammenhang Chloride, Iodide, Sulfate, Hydrogensulfate, Karbonate, Hydrogenkarbonate, Phosphate, Dihydrogenphosphate oder Hydrogenphosphate der Alkali- und Erdalkalimetalle. Ganz besonders bevorzugt umfasst die Hydrogelmatrix Natriumchlorid, Kaliumchlorid, Magnesiumchlorid, Kalziumchlorid oder Mischungen hiervon. Diese Salze simulieren in besonders guter Weise das Elektrolyt-Gemisch in einem von einer Wunde abgegebenen Wundserum. Damit stellt eine diese Salze umfassende Hydrogelmatrix einer Wunde ein besonders wundheilungsförderndes Klima zur Verfügung.

Hierbei kann vorgesehen sein, dass die Hydrogelmatrix 0 bis 5 Gew.-% mindestens eines Salzes umfasst. Insbesondere umfasst die Hydrogelmatrix 0,1 bis 3 Gew.-% eines Salzes und ganz besonders bevorzugt 0,5 bis 1,5 Gew.-% eines Salzes.

Insgesamt kann gemäß der vorliegenden Erfindung vorgesehen sein, dass die wasserhaltige Hydrogelmatrix bevorzugt mindestens 20 Gew.-% Wasser und mindestens 10 Gew.-% Polyurethan-Polyharnstoff-Copolymer umfasst. Eine weiterhin bevorzugte Hydrogelmatrix umfasst mindestens 20 Gew.-% Wasser und mindestens 15 Gew.-% Polyurethan-Polyharnstoff-Copolymer. Hierbei kann weiterhin bevorzugt vorgesehen sein, dass die Hydrogelmatrix aus 6 bis 60 Gew.-% eines Präpolymers mit aliphatischen Diisocyanat-Gruppen, 4 bis 40 Gew.-% Polyamin auf Polyethylenoxidbasis, 0 bis 50 Gew.-% eines mehrwertigen Alkohols, 0 bis 5 Gew.-% mindestens eines Salzes ausgewählt aus der Gruppe Natriumchlorid, Kaliumchlorid, Magnesiumchlorid, Kalziumchlorid oder Mischungen hiervon und mindestens 20 Gew.-% Wasser gebildet wird.

Weiterhin bevorzugt kann vorgesehen sein, dass die Hydrogelmatrix aus 6 bis 30 Gew.-% eines Präpolymers mit aliphatischen Diisocyanat-Gruppen, 4 bis 20 Gew.-% Diamin auf Polyethylenoxidbasis, 10 bis 30 Gew.-% eines mehrwertigen Alkohols ausgewählt aus der Gruppe bestehend aus Propylenglykol und/ oder Glycerin, 0,5 - 1,5 Gew.-% eines Salzes ausgewählt aus der Gruppe Natriumchlorid, Kaliumchlorid, Magnesiumchlorid, Kalziumchlorid oder Mischungen hiervon und mindestens 30 Gew.-% Wasser gebildet wird.

Ganz besonders bevorzugt kann vorgesehen sein, dass die Hydrogelmatrix aus 6 bis 20 Gew.-% Präpolymer mit Isophorondiisocyanat-Enden, 4 bis 15 Gew.-% Diamin auf Polyethylenoxidbasis, 15 bis 20 Gew.-% Polypropylenglycol und/oder Glycerin, 0,5 bis 1,5 Gew.-% eines Salzes ausgewählt aus der Gruppe Natriumchlorid, Kaliumchlorid, Magnesiumchlorid, Kalziumchlorid oder Mischungen hiervon und mindestens 40 Gew.-% Wasser gebildet wird. Diese Hydrogelmatrix weist eine freie Absorption A2 (bezogen auf die wasserenthaltende Hydrogelmatrix) von mindestens 1 g/g und höchstens 5 g/g auf, stellt ein nicht reizendes, flüssigkeitsabsorbierendes, vor Bakterien schützendes, polsterndes, hautartiges Medium bereit und ist somit besonders gut als Wundkontaktschicht geeignet.

In einer bevorzugten Ausführungsform umfasst die Hydrogelmatrix 37 bis 43 Gew.-% Propylenglykol, ein Vorpolymer mit Isophorondiisocyanatendgruppen (im Folgenden als "Isocyanat" bezeichnet) und ein Diamin auf Polyethylenoxidbasis in einer Menge von insgesamt 12 bis 16,5 Gew.-%, 0 bis 5 Gew.-% eines anorganischen Chlorids, sowie einen Rest Wasser, wobei das Verhältnis der reaktiven Gruppen von Isocyanat zu den Amingruppen des Diamins 1,25 bis 1,35 betragen soll.

Um die erwünschten Eigenschaften der wasserhaltigen Hydrogelmatrix zu erhalten, ist es vorteilhaft, dass die Zusammensetzung der Hydrogelmatrix einerseits einen Propylenglykolanteil von 37 bis 43 Gew.-% umfasst. Dies ist im Vergleich zu den im Stand der Technik üblicherweise beschriebenen Zusammensetzungen ein wesentlich höherer Gehalt an Propylenglykol. Andererseits kann die Zusammensetzung ein Vorpolymer mit Isophorondiisocyanatendgruppen und ein Diamin auf Polyethylenoxidbasis in einer Menge von insgesamt 12 bis 16,5 Gew.-% umfassen, wobei ein auf den Bereich von 1,25 bis 1,35 eingestelltes Verhältnis ("NCO:NH2") der reaktiven Gruppen von Isocyanat ("NCO") zu den Amingruppen des Diamins ("NH2") vorliegen kann. Weiterhin umfasst die Hydrogelmatrix 0 bis 5 Gew.-% mindestens eines anorganischen Chlorids. Es wurde gefunden, dass bei einer Abweichung von der in der Erfindung offenbarten Zusammensetzung ein Hydrogel mit vergleichsweise ungünstigeren Eigenschaften erhalten wird, beispielsweise ein Hydrogel mit einem Tack, welches mit einem Wert von weniger als 3 oder mehr als 4 eingestuft wurde, ein Hydrogel mit einem unerwünscht hohem pH-Wert von mehr als 8 oder ein Hydrogel, welches nicht ausreichend aushärtet.

Besonders vorteilhafte Hydrogele können erhalten werden, wenn die Zusammensetzung 37 bis 43 Gew.-% Propylenglykol, vorzugsweise 39 bis 41 Gew.-% Propylenglykol und ein Vorpolymer mit Isophorondiisocyanatendgruppen und ein Diamin auf Polyethylenoxidbasis in einer Menge von insgesamt 12 bis 16,5 Gew.-%, vorzugsweise 14 bis 16 Gew.-%, umfasst, wobei ein auf den Bereich von 1,27 bis 1,33, idealerweise auf 1,29 bis 1,31, eingestelltes Verhältnis der reaktiven Gruppen von Isocyanat zu den Amingruppen des Diamins vorliegt. Derartige Hydrogele weisen hervorragende Eigenschaften hinsichtlich Tack, pH-Wert und Stabilität auf.

Als sehr günstig hat sich dabei erwiesen, wenn die Hydrogelmatrix 0,5 bis 1,5 Gew.-% eines anorganischen Chlorids umfasst, wobei es sich bei dem anorganischen Chlorid vorzugsweise um Natriumchlorid handelt. Insbesondere umfasst die Hydrogelmatrix 0,9 Gew.-% Natriumchlorid.

In einer Ausführungsform hat das Hydrogel einen Wassergehalt von mindestens 50 Gew.-% bezogen auf das Gesamtgewicht des Hydrogels, wobei das Hydrogel mindestens ein gelbildendes Polysaccharid, mindestens ein Acrylsäure-Derivat und ein Elektrolyt-Gemisch umfasst, wobei das Elektrolyt-Gemisch mindestens zwei verschiedene Elektrolyte aufweist.

Gemäß einer Ausführungsform umfasst das Hydrogel vorzugsweise mindestens ein gelbildendes Polysaccharid ausgewählt aus der Gruppe der Cellulose-Derivate oder deren Salze, Alginate oder deren Derivate, Chitin oder dessen Derivate oder dessen Salze oder Stärke. Hierbei ist der Ursprung der gelbildenden Polysaccharide unbeachtlich, das heißt, dass diese gelbildenden Polysaccharide pflanzlichen oder tierischen Ursprungs sein können oder auf synthetischem Weg, beispielsweise durch mikrobiologische Verfahren, hergestellt sein können. Es ist auch möglich Polysaccharide zu verwenden, die pflanzlichen oder tierischen Ursprungs und durch chemische Synthese modifiziert sind.

Des Weiteren ist vorgesehen, dass das Hydrogel eine dynamische Viskosität von 5.000 bis 60.000 mPa s, insbesondere 5.000 bis 50.000 mPa s und ganz besonders 10.000 bis 40.000 mPa s aufweist (gemessen mittels Bohlin-Rheometer Typ CSR - 10, Kegelspindel 4° / Ø 40 mm, Spaltabstand 100 $\mu$m, Oszillometrische Messung, T= 22-27 °C). Ein solches Hydrogel lässt sich besonders gut und gleichmäßig beispielsweise mit einem Spachtel über und in einer Wunde verteilen, weist auch bei der Aufnahme von Wundexsudat einen guten Zusammenhalt auf und fließt nicht aus einer zu behandelnden Wunde.

In einer besonders bevorzugten Ausführungsform umfasst das Hydrogel mindestens ein gelbildendes Polysaccharid und ein Acrylsäure-Derivat, wobei das Gewichtsverhältnis des oder der Polysaccharide zu dem Acrylsäure-Derivat in dem Hydrogel dem Verhältnis von 20:1 bis 1:1, insbesondere 15:1 bis 1:1 und ganz besonders 10:1 bis 1:1 entspricht.

In einer besonders bevorzugten Ausführungsform weist das Hydrogel eine Leitfähigkeit von mindestens 4000 $\mu$S cm$^{-1}$ insbesondere mindestens 6000 $\mu$S cm$^{-1}$ und ganz besonders bevorzugt 6000-20000 $\mu$S cm$^{-1}$ auf. Die Leitfähigkeit ist insbesondere durch die Menge an Elektrolyt-Gemisch einzustellen. Dieses Einstellen ist vorteilhaft, da die Leitfähigkeit eines Hydrogels von den Komponenten des Gels und ihren Konzentrationen abhängt. Beispielsweise ist die Leitfähigkeit abhängig von der Art und der Konzentration des eingesetzten gelbildenden Polymers oder von der Art und der Konzentration des eingesetzten Polyols. Diese Komponenten setzten in unterschiedlichem Maß die Leitfähigkeit eines Hydrogels im Vergleich zu einer reinen Salzlösung herunter. Somit muss die Menge an Elektrolyt-Gemisch individuell an die übrigen Inhaltsstoffe des Gels angepasst werden, um eine bestimmte Leitfähigkeit einzustellen.

In einer Ausführungsform ist die Wundauflage als Inselverband ausgestaltet und die Schicht des adhäsiven und sterilen Silicons 2 ist zwischen der Deckschicht und der Hydrogelschicht angeordnet und überragt die Hydrogelschicht, so dass die Schicht des adhäsiven und sterilen Silicons als Haftmittel für die Wundauflage dient. Das erfindungsgemäße Verfahren ermöglicht es weiterhin, die Klebe- und Adhäsionseigenschaften des Silicons 2 derart einzustellen, dass das Silicon 2 eine Initialhaftung (sog. Tack) entfaltet. Eine derartige Initialhaftung fixiert eine auf die Haut bzw. Wundfläche aufgebrachte Wundauflage so lange, bis weitere (sekundäre) Verbandsmittel (z.B. Mullbinde, Klebefolie etc.) aufgebracht wurden, über welche die endgültige Befestigung der Wundauflage eingestellt wird. Dies kann von Vorteil sein, bei entzündeten Wundrändern oder Wunden, die einen häufigen Verbandswechsel erfordern. Die spätere Ablösung der Wundauflage ist aufgrund der Initialhaftung vereinfacht und weniger schmerzhaft für den Patienten. Demgemäß umfasst die vorliegende Erfindung auch Wundauflagen mit Initialhaftung, wobei die Initialhaftung durch das Silicon 2 in der Wundauflage gewährleistet wird. Insbesondere niedrige Adhäsionskräfte im Bereich von 0,05-7,5 N/cm$^2$ können für eine Initialhaftung verwendet werden, wobei die Größe der Haftfläche einer Wundauflage zu berücksichtigen ist.

In einer Ausführungsform weist das Hydrogel eine freie Absorption A$_3$ (gemessen nach DIN EN 13726-1 (2002)) von mindestens 1 g/g und höchstens 10 g/g auf. Die Hydrogelschicht hat vorzugsweise einen pH-Wert von 5-8, um den Heilungsprozess zu verbessern. Die Hydrogelschicht umfasst vorzugsweise ein Puffersystem. Durch Verwendung eines Puffersystems kann der pH-Wert nahezu konstant gehalten werden. Die Hydrogelschicht hat bevorzugt eine Schichtdicke von 3,2-5,9 mm.

Die Hydrogelschicht kann ferner antimikrobielle Substanzen und/oder Infektionsindikatoren umfassen.

Die Wundauflage kann weitere optionale Schichten umfassen. So kann die Wundauflage eine Schaumstoffschicht und/oder eine Klebeschicht umfassen.

In einer Ausführungsform betrifft die vorliegende Erfindung ein Verfahren zur Herstellung der erfindungsgemäßen Wundauflage, umfassend

(a) Bereitstellen einer Anordnung umfassend eine Schicht umfassend ein Silicon 1 sowie eine Deckschicht und/oder eine Hydrogelschicht,
(b) optional Verpacken der Anordnung in einer Verpackung,
(c) Bestrahlung der Anordnung mit ionisierender Strahlung zur Härtung und Sterilisierung mit einer Energiedosis E von 5-60 kGy,

wobei das Silicon 1

(a) erhältlich ist durch Polykondensation einer Zusammensetzung umfassend Dimethyldichlorsilan und/oder Trimethylmonochlorsilan sowie Einheiten aus der Gruppe bestehend aus

(i) Monomethyltrichlorsilan, und/oder
(ii) einem Element der Gruppe bestehend aus Maleinsäure, Fumarsäure und Trans-3-Hexendisäure sowie davon.

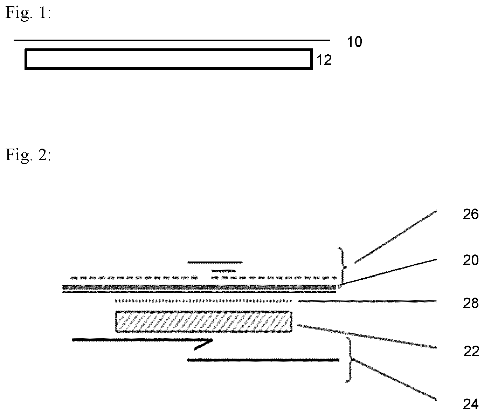

Der Begriff "Anordnung" bezieht sich auf eine vorbestimmte Abfolge von Schichten. Eine Anordnung kann die erfindungsgemäße Zusammensetzung enthalten. Daneben können weitere Schichten enthalten sein. Hierzu gehören a) eine Wundkontaktschicht umfassend oder bestehend aus einer Hydrogelschicht und / oder einer erfindungsgemäßen Siliconschicht, b) eine Zwischenschicht, c) eine Deckschicht und d) eine Klebeschicht. Die Deckschicht sollte der Wundkontaktschicht immer entgegengesetzt positioniert sein. Beispielhafte Anordnungen lassen sich Fig. 1 und Fig. 2 entnehmen.

In einer Ausführungsform findet das Verpacken und die Bestrahlung auf einer Auflagefläche eines Bewegungselements, insbesondere eines Förderbandes, statt.

In einer bevorzugten Ausführungsform ist das erfindungsgemäße Verfahren ein kontinuierliches Verfahren. Durch die Sterilisation durch Bestrahlung auf einer Auflagefläche eines Bewegungselementes wird die Wundauflage effizienter produziert. Zu diesem Zweck kann das Bewegungselement angehalten werden oder mit einer vorbestimmten Geschwindigkeit durch eine Strahlungsquelle laufen.

Insbesondere werden Kosten durch ein solches "Inline-Verfahren" reduziert und das Verfahren insgesamt effizienter gestaltet. Somit kann die Konfektionierung, Verpackung, Vernetzung und Sterilisierung in einem Prozessschritt durchgeführt werden.

In diesem Sinne umfasst das erfindungsgemäße Verfahren auch ein automatisiertes oder teilweise automatisiertes Verfahren, das ein oder mehrere Merkmale des genannten "Inline-Verfahrens" umfassen kann.

Weiterhin betrifft die vorliegende Erfindung die Verwendung eines Silicons 1 in einer Wundkontaktschicht, wobei das Silicon 1 seine adhäsiven Eigenschaften während einer Sterilisierung mit ionisierender Strahlung zur Härtung und Sterilisierung mit einer Energiedosis E von 5-60 kGy beibehält, und wobei das Silicon 1

(a) erhältlich ist durch Polykondensation einer Zusammensetzung umfassend Dimethyldichlorsilan und/oder Trimethylmonochlorsilan sowie Einheiten aus der Gruppe bestehend aus

(i) Monomethyltrichlorsilan, und/oder
(ii) einem Element der Gruppe bestehend aus Maleinsäure, Fumarsäure und Trans-3-Hexendisäure sowie Mischungen davon.

**Experimenteller Teil**

**Analytik:** Tacktest zur Bestimmung der maximalen Adhäsionskraft $F_{max}$

Die Adhäsionskraft des Silicons wurde auf Stahl getestet. Zum Einsatz kam eine Zugprüfmaschine, welche dem Standard DIN EN ISO 7500-01 entsprach. Sämtliche Messungen fanden unter standardisierten Bedingungen von 23°C und 50 % relativer Luftfeuchtigkeit statt. Die Proben wurden an einem horizontal beweglichen Träger mittels doppelseitigen Klebebands befestigt. Zur Durchführung der Messung wurde ein Metallgewicht mit einer Gewichtskraft von 0,245 N mit einer Unterseite aus Glas verwendet. Die Unterseite wurde vor Beginn der Messung mit einem Ethanolgetränkten Pad gereinigt. Das Gewicht wurde mit der Unterseite voran auf dem Silicon platziert und die Messung gemäß den Parametern in Tabelle 1 durchgeführt:

Tabelle 1

| Anfangsgeschwindigkeit [mm / min] | Abziehgeschwindigkeit [mm / min] | Kontaktzeit [s] |
|---|---|---|
| 100 | 400 | 2 |

Nach Ablauf der Kontaktzeit wurde mittels der Zugprüfmaschine die zum Abziehen des Gewichts in einem 90°-Winkel notwendige Kraft gemessen. Pro Probe/Zusammensetzung (siehe unten) wurde jeweils dreimal die maximale Adhäsionskraft bestimmt und dann der Mittelwert gebildet und auf eine Stelle nach dem Komma gerundet.

**Beispiele:** Herstellung von Silicon 2

Das Silicon 2 wurde mit verschiedenen Zusammensetzung hergestellt, wobei die Herstellung gemäß der untenstehenden Schritte erfolgt:

- Dichlordimethylsilan und Trichlormethylsilan und gegebenenfalls Chlortrimethylsilan wurden in einem Becherglas gemischt.

- Gegebenenfalls wurde Ether (1,4 Butandiol Divinylether) zu der Silanmischung gegeben
- Anschließend wurde destilliertes Wasser oder die wässrige Säurelösung (2 mg Maleinsäure in 100 ml Wasser oder 0,1 mg in 100 ml Wasser) tröpfchenweise zu der Mischung gegeben, wobei eine exotherme Reaktion mit Gas- und Bläschenbildung einsetzte.
- Die wässrige Siliconphase wurde mithilfe einer Pasteurpipette von den nach der exothermen Reaktion gebildeten zwei Phasen abgetrennt und in eine Petrischale überführt.
- Nach trocknen lassen wurde das Silicon mit Hilfe eines Spatels in einer dünnen Schicht auf einen PU Film aufgetragen und die entsprechenden Proben wurden keiner Bestrahlung oder Bestrahlung verschiedener Stärke ausgesetzt.
- Messen der $F_{max}$ der verschiedenen Proben durch den oben beschriebenen Tacktest

Die verschiedenen Zusammensetzungen, die Bestrahlungsstärke und die maximale Adhäsionskraft sind in untenstehender Tabelle 2 aufgeführt.

**Tabelle 2**

| Bezeichnung/ Nummer | Dichlordimethylsilan [ml] | Trichlormethylsilan [ml] | reines dest. Wasser [ml] | Maleinsäurelösung (2g Maleinsäure in 100ml Wasser) [ml] | Fumarsäurelösung (0,1g Fumarsäure in 100ml Wasser) [ml] | Ether [ml] | Chlortrimethylsilan [ml] | Fmax bei 5 kGy [N] | Fmax bei 15 kGy [N] | Fmax bei 25 kGy [N] | Fmax ohne Bestrahlung [N] |
|---|---|---|---|---|---|---|---|---|---|---|---|
| 1 | 5 | 1 | 2 | | - | - | - | 0,1 | 0,1 | 0,3 | 0,2 |
| 2 | 7 | 1 | 2 | | - | - | - | 0,8 | 1,3 | 0,3 | 0,8 |
| 3 | 9 | 1 | 3 | | - | - | - | 2,2 | 1,3 | 1,9 | 0,0 |
| 4 | 5 | 1 | - | 2 | | - | - | 0,0 | 1,1 | 0,1 | 0,1 |
| 5 | 7 | 1 | - | 2 | | - | - | 0,1 | 0,6 | 0,2 | 0,1 |
| 6 | 7 | 1 | - | 3 | | - | - | 0,6 | 0,3 | 0,3 | 0,3 |
| 7 | 7 | 1 | - | 4 | | - | - | 1,8 | 2,5 | 0,7 | 2,2 |
| 8 | 9 | 1 | - | 2 | | - | - | 0,1 | 0,4 | 0,0 | 0,1 |
| 9 | 7 | 1 | - | 3 | | 1 | - | 1,2 | 0,8 | 0,5 | 0,1 |
| 10 | 7 | 1 | - | 3 | | 2 | - | 0,4 | 0,5 | 1,0 | 0,5 |
| 11 | 7 | 1 | - | 3 | | 3 | - | 2,6 | 1,3 | 0,8 | 0,2 |
| 12 | 7 | 1 | - | | 4 | - | - | 1,0 | 0,5 | 0,1 | 0,9 |
| 13 | 7 | 1 | - | | 3 | 3 | - | 1,2 | 3,5 | 2,3 | 0,0 |
| 14 | 9 | 1 | 3 | | - | - | 1 | 0,0 | 0,0 | 0,0 | 0,0 |
| 15 | 9 | 1 | 3 | | - | - | 2 | 0,0 | 0,0 | 0,0 | 1,5 |
| 16 | 9 | 1 | 3 | | - | - | 3 | 0,0 | 0,0 | 0,0 | 0,0 |

Wie aus der oben aufgeführten Tabelle ersichtlich, kann ein Silicon mit adhäsiven Eigenschaften hergestellt werden, das mit einer für die Sterilisation benötigten Strahlung kompatibel ist.

**Patentansprüche**

1. Verfahren zur Herstellung eines adhäsiven und sterilen Silicons 2, umfassend Bestrahlung eines Silicons 1 mit ionisierender Strahlung mit einer Energiedosis von 5-60 kGy, wobei das Silicon 1

   (a) erhältlich ist durch Polykondensation einer Zusammensetzung umfassend Dimethyldichlorsilan und/oder Trimethylmonochlorsilan sowie Einheiten aus der Gruppe bestehend aus

   (i) Monomethyltrichlorsilan, und/oder
   (ii) einem Element der Gruppe bestehend aus Maleinsäure, Fumarsäure und Trans-3-Hexendisäure sowie Mischungen davon.

2. Verfahren zur Herstellung eines adhäsiven und sterilen Silicons 2 nach Anspruch 1, wobei das Silicon 1 und/oder die Zusammensetzung gemäß (a) weiterhin ein Element umfasst aus der Gruppe bestehend aus
   (iii) Divinylether, 1,4-Butandiol Divinylether, Diethylenglykol-Divinylether und Triethylenglykol-Divinylether sowie Mischungen davon.

3. Verfahren zur Herstellung eines adhäsiven und sterilen Silicons 2 nach Anspruch 1 oder 2, wobei das Silicon 1 einen Vinylgehalt von 0,1-10 Gew.-% aufweist.

4. Verfahren zur Herstellung eines adhäsiven und sterilen Silicons 2 nach einem der Ansprüche 1-3, wobei die Zusammensetzung 0,1-10 Mengen-% Monomethyltrichlorsilan umfasst, und die Energiedosis 5-60 kGy, bevorzugt 5-45 kGy, besonders bevorzugt 10-20 kGy, beträgt.

5. Verfahren zur Herstellung eines adhäsiven und sterilen Silicons 2 nach einem der Ansprüche 1-4, wobei die Zusammensetzung 0,1-10 Mengen-% eines Elements der Gruppe bestehend aus Divinylether, 1,4-Butandiol Divinylether, Diethylenglykol-Divinylether und Triethylenglykol-Divinylether sowie Mischungen davon umfasst, und die Energiedosis 5-60 kGy, bevorzugt 15-45 kGy, besonders bevorzugt 20-40 kGy, beträgt, und/oder wobei das Silicon 2 eine Adhäsionskraft von 0,4-4 N/cm$^2$ aufweist, gemessen wie in der Beschreibung beschrieben, und gemäß Eur. Ph. 9.00 /2.06.01.00 steril ist.

6. Verfahren zur Herstellung eines adhäsiven und sterilen Silicons 2 nach einem der Ansprüche 1-5, wobei das Silicon 1 weiterhin ausgewählt ist aus der Gruppe bestehend aus Vinyl-Methyl-Silicon, Phenyl-Vinyl-Methyl-Silicon und Fluor-Vinyl-Methyl-Silicon.

7. Adhäsives und steriles Silicon 2 erhältlich durch das Verfahren nach einem der Ansprüche 1-6.

8. Silicon-Schicht enthaltend adhäsives und steriles Silicon nach Anspruch 7.

9. Wundauflage umfassend eine Silicon-Schicht nach Anspruch 8, wobei die Silicon-Schicht als Wundkontaktschicht ausgestaltet sein kann.

10. Wundauflage nach Anspruch 9, ferner umfassend eine Hydrogelschicht auf einer Wundkontaktschicht, wobei die Hydrogelschicht als Hydrogelmatrix ausgebildet sein kann.

11. Wundauflage nach Anspruch 10, wobei die Wundauflage als Inselverband ausgestaltet ist und wobei die Schicht des adhäsiven und sterilen Silicons 2 zwischen einer Deckschicht und der Hydrogelschicht angeordnet ist und die Hydrogelschicht überragt, so dass die Schicht des adhäsiven und sterilen Silicons als Haftmittel für die Wundauflage dient.

12. Verfahren zur Herstellung der Wundauflage nach einem der Ansprüche 9-11, umfassend

    (a) Bereitstellen einer Anordnung umfassend eine Schicht umfassend ein Silicon 1, sowie eine Deckschicht und/oder eine Hydrogelschicht,

(b) optional Verpacken der Anordnung in einer Verpackung,

(c) Bestrahlung der Anordnung mit ionisierender Strahlung zur Härtung und Sterilisierung mit einer Energiedosis E von 5-60 kGy,

wobei das Silicon 1

(a) erhältlich ist durch Polykondensation einer Zusammensetzung umfassend Dimethyldichlorsilan und/oder Trimethylmonochlorsilan sowie Einheiten aus der Gruppe bestehend aus

(i) Monomethyltrichlorsilan, und/oder
(ii) einem Element der Gruppe bestehend aus Maleinsäure, Fumarsäure und Trans-3-Hexendisäure sowie Mischungen davon.

13. Verfahren nach Anspruch 12, wobei das Silicon 1 weiterhin ausgewählt ist aus der Gruppe bestehend aus Vinyl-Methyl-Silicon, Phenyl-Vinyl-Methyl-Silicon und Fluor-Vinyl-Methyl-Silicon.

14. Verwendung eines Silicons 1 in einer Wundkontaktschicht, wobei das Silicon 1 seine adhäsiven Eigenschaften während einer Sterilisierung mit ionisierender Strahlung zur Härtung und Sterilisierung mit einer Energiedosis E von 5-60 kGy beibehält, und wobei das Silicon 1

(a) erhältlich ist durch Polykondensation einer Zusammensetzung umfassend Dimethyldichlorsilan und/oder Trimethylmonochlorsilan sowie Einheiten aus der Gruppe bestehend aus

(i) Monomethyltrichlorsilan, und/oder
(ii) einem Element der Gruppe bestehend aus Maleinsäure, Fumarsäure und Trans-3-Hexendisäure sowie Mischungen davon.

15. Verwendung nach Anspruch 14, wobei das Silicon 1 weiterhin ausgewählt ist aus der Gruppe bestehend aus Vinyl-Methyl-Silicon, Phenyl-Vinyl-Methyl-Silicon und Fluor-Vinyl-Methyl-Silicon.

**Claims**

1. A process for the preparation of an adhesive and sterile silicone 2, comprising irradiating a silicone 1 with ionizing radiation with an absorbed dose of 5-60 kGy, wherein the silicone 1

(a) is obtainable by polycondensation of a composition comprising dimethyldichlorosilane and/or trimethylmonochlorosilane and units selected from the group consisting of

(i) monomethyltrichlorosilane, and/or
(ii) an element from the group consisting of maleic acid, fumaric acid and trans-3-hexenedioic acid, and mixtures thereof.

2. The process for the preparation of an adhesive and sterile silicone 2 according to claim 1, wherein the silicone 1 and/or the composition according to (a) further comprises an element from the group consisting of
(iii) divinyl ether, 1,4-butanediol divinyl ether, diethylene glycol divinyl ether and triethylene glycol divinyl ether and mixtures thereof.

3. The process for the preparation of an adhesive and sterile silicone 2 according to claim 1 or 2, wherein the silicone 1 has a vinyl content of 0. 1-10% by weight.

4. The process for the preparation of an adhesive and sterile silicone 2 according to any one of claims 1-3, wherein the composition comprises 0.1-10 volume % of monomethyltrichlorosilane, and the absorbed dose is 5-60 kGy, preferably 5-45 kGy, particularly preferably 10-20 kGy.

5. The process for the preparation of an adhesive and sterile silicone 2 according to any one of claims 1-4, wherein the composition comprises 0.1-10 volume % of an element of the group consisting of divinyl ether, 1,4-butanediol divinyl ether, diethylene glycol divinyl ether and triethylene glycol divinyl ether, and mixtures thereof, and the absorbed dose is 5-60 kGy, preferably 15-45 kGy, particularly preferably 20-40 kGy, and/or wherein the silicone 2 has an adhesion force of 0.4-4 $N/cm^2$, measured as described in the description, and is sterile according to Eur. Ph. 9.00/2.06.01.00.

6. The process for the preparation of an adhesive and sterile silicone 2 according to any one of claims 1-5, wherein the silicone 1 is further selected from the group consisting of vinyl-methyl silicone, phenyl-vinyl-methyl silicone and fluoro-vinyl-methyl silicone.

7. An adhesive and sterile silicone 2 obtainable by the process according to any one of claims 1-6.

8. A silicone layer comprising an adhesive and sterile silicone according to claim 7.

9. A wound dressing comprising a silicone layer according to claim 8, wherein the silicone layer can be designed as a wound contact layer.

10. The wound dressing according to claim 9, further comprising a hydrogel layer on a wound contact layer, wherein the hydrogel layer can be designed as a hydrogel matrix.

11. The wound dressing according to claim 10, wherein the wound dressing is designed as an island dressing and wherein the layer of adhesive and sterile silicone 2 is arranged between a cover layer and the hydrogel layer and projects beyond the hydrogel layer, so that the layer of adhesive and sterile silicone serves as a adhesive for the wound dressing.

12. A method for the preparation of the wound dressing according to any one of claims 9-11, comprising

(a) providing an assembly comprising a layer comprising a silicone 1, and a cover layer and/or a hydrogel layer,
(b) optionally packaging the assembly in a package,
(c) irradiating the assembly with ionizing radiation for curing and sterilization with an absorbed dose E of 5-60 kGy,

wherein the silicone 1

(a) is obtainable by polycondensation of a composition comprising dimethyldichlorosilane and/or trimethylmonochlorosilane and units selected from the group consisting of

(i) monomethyltrichlorosilane, and/or
(ii) an element of the group consisting of maleic acid, fumaric acid and trans-3-hexenedioic acid and mixtures thereof.

13. The method according to claim 12, wherein the silicone 1 is further selected from the group consisting of vinyl-methyl silicone, phenyl-vinyl-methyl silicone and fluoro-vinyl-methyl silicone.

14. Use of a silicone 1 in a wound contact layer, wherein the silicone 1 retains its adhesive properties during sterilization with ionizing radiation for curing and sterilization with an absorbed dose E of 5-60 kGy, and wherein the silicone 1

(a) is obtainable by polycondensation of a composition comprising dimethyldichlorosilane and/or trimethylmonochlorosilane and units selected from the group consisting of

(i) monomethyltrichlorosilane, and/or
(ii) an element of the group consisting of maleic acid, fumaric acid and trans-3-hexenedioic acid and mixtures thereof.

15. The use according to claim 14, wherein the silicone 1 is further selected from the group consisting of vinyl-methyl-silicone, phenyl-vinyl-methyl-silicone and fluoro-vinyl-methyl-silicone.

**Revendications**

**1.** Procédé de fabrication d'une silicone 2 adhésive et stérile, comprenant l'exposition d'une silicone 1 à un rayonnement ionisant avec une dose absorbée de 5-60 kGy, dans lequel la silicone 1

    (a) peut être obtenue par polycondensation d'une composition comprenant du diméthyldichlorosilane et/ou du triméthylmonochlorosilane ainsi que des unités du groupe constitué

        (i) du monométhyltrichlorosilane, et/ou
        (ii) d'un élément du groupe constitué de l'acide maléique, de l'acide fumarique et de l'acide trans-3-hexènedioïque ainsi que de mélanges de ceux-ci.

**2.** Procédé de fabrication d'une silicone adhésive et stérile 2 selon la revendication 1, dans lequel la silicone 1 et/ou la composition selon (a) comprend en outre un élément du groupe constitué
    (iii) d'éther divinylique, d'éther divinylique de 1,4-butanediol, d'éther divinylique de diéthylèneglycol et d'éther divinylique de triéthylèneglycol ainsi que de mélanges de ceux-ci.

**3.** Procédé de fabrication d'une silicone adhésive et stérile 2 selon la revendication 1 ou 2, dans lequel la silicone 1 a une teneur en vinyle de 0,1 à 10 % en poids.

**4.** Procédé de fabrication d'une silicone adhésive et stérile 2 selon l'une des revendications 1 à 3, dans lequel la composition comprend 0,1 à 10 % en volume de monométhyltrichlorosilane, et la dose absorbée est de 5 à 60 kGy, de manière préférée de 5 à 45 kGy, de manière particulièrement préférée de 10 à 20 kGy.

**5.** Procédé de fabrication d'une silicone adhésive et stérile 2 selon l'une des revendications 1 à 4, dans lequel la composition comprend de 0,1 à 10 % en volume d'un élément du groupe constitué d'éther divinylique, d'éther divinylique de 1,4-butanediol, d'éther divinylique de diéthylèneglycol et d'éther divinylique de triéthylèneglycol ainsi que de mélanges de ceux-ci, et la dose absorbée est de 5 à 60 kGy, de manière préférée de 15 à 45 kGy, de manière particulièrement préférée de 20 à 40 kGy, et/ou dans lequel la silicone 2 a un pouvoir adhésif de 0,4 à 4 N/cm$^2$, mesuré tel que décrit dans la description, et est stérile selon Eur. Ph. 9.00 /2.06.01.00.

**6.** Procédé de fabrication d'une silicone 2 adhésive et stérile selon l'une des revendications 1 à 5, dans lequel la silicone 1 est en outre choisie parmi le groupe constitué de vinyl-méthyl-silicone, de phényl-vinyl-méthyl-silicone et de fluoro-vinyl-méthyl-silicone.

**7.** Silicone adhésive et stérile 2 pouvant être obtenue par le procédé selon l'une des revendications 1 à 6.

**8.** Couche de silicone contenant de la silicone adhésive et stérile selon la revendication 7.

**9.** Pansement comprenant une couche de silicone selon la revendication 8, dans lequel la couche de silicone peut être formée comme une couche en contact avec la plaie.

**10.** Pansement selon la revendication 9, comprenant en outre une couche d'hydrogel sur une couche en contact avec la plaie, dans lequel la couche d'hydrogel peut être formée comme une matrice d'hydrogel.

**11.** Pansement selon la revendication 10, dans lequel le pansement est forme comme un pansement à îlot et dans lequel la couche de silicone 2 adhésive et stérile est disposée entre une couche de recouvrement et la couche d'hydrogel et la couche d'hydrogel dépasse de sorte que la couche de silicone adhésive et stérile sert d'adhésif pour le pansement.

**12.** Procédé de fabrication du pansement selon l'une des revendications 9 à 11, comprenant les étapes consistant à :

    (a) fournir un ensemble comprenant une couche comprenant une silicone 1 ainsi qu'une couche de recouvrement et/ou une couche d'hydrogel,
    (b) emballer facultativement l'ensemble dans un emballage,
    (c) exposer l'ensemble à un rayonnement ionisant pour le durcissement et la stérilisation avec une dose absorbée E de 5 à 60 kGy,

dans lequel la silicone 1

(a) peut être obtenue par polycondensation d'une composition comprenant du diméthyldichlorosilane et/ou du triméthylmonochlorosilane ainsi que des unités du groupe constitué

(i) du monométhyltrichlorosilane, et/ou
(ii) d'un élément du groupe constitué de l'acide maléique, de l'acide fumarique et de l'acide trans-3-hexènedioïque ainsi que de mélanges de ceux-ci.

13. Procédé selon la revendication 12, dans lequel la silicone 1 est en outre choisie parmi le groupe constitué de vinyl-méthyl-silicone, de phényl-vinyl-méthyl-silicone et de fluoro-vinyl-méthyl-silicone.

14. Utilisation d'une silicone 1 dans une couche en contact avec une plaie, dans laquelle la silicone 1 conserve ses propriétés adhésives pendant une stérilisation avec un rayonnement ionisant pour le durcissement et la stérilisation avec une dose absorbée E de 5 à 60 kGy, et dans laquelle la silicone 1

(a) peut être obtenue par polycondensation d'une composition comprenant du diméthyldichlorosilane et/ou du triméthylmonochlorosilane ainsi que des unités du groupe constitué

(i) du monométhyltrichlorosilane, et/ou
(ii) d'un élément du groupe constitué de l'acide maléique, de l'acide fumarique et de l'acide trans-3-hexènedioïque ainsi que de mélanges de ceux-ci.

15. Utilisation selon la revendication 14, dans laquelle la silicone 1 est en outre choisie parmi le groupe constitué de vinyl-méthyl-silicone, de phényl-vinyl-méthyl-silicone et de fluoro-vinyl-méthyl-silicone.

EP 4 217 436 B1

Fig. 1:

10
12

Fig. 2:

26
20
28
22
24

19

**IN DER BESCHREIBUNG AUFGEFÜHRTE DOKUMENTE**

**In der Beschreibung aufgeführte Patentdokumente**

- US 2020146900 A1

**In der Beschreibung aufgeführte Nicht-Patentliteratur**

- **C. HARZDORF.** Bestimmung von Si-H und Si-Vinyl in siliciumorganischen Substanzen. *Zeitschrift für Analytische Chemie,* 1975, vol. 276, 279-283